# EUROPÄISCHE PATENTSCHRIFT

(11) **EP 0 456 871 B1**
(45) Veröffentlichungstag und Bekanntmachung des Hinweises auf die Patenterteilung: **14.04.1993**
(21) Anmeldenummer: 90109422.7
(22) Anmeldetag: 18.05.1990
(51) Int. Cl.: A61M 5/168

(54) **Vorrichtung und Verfahren zum Ermitteln und Regeln der Anzahl von eine Tropfenkammer durchlaufenden Tropfen**
Device and procedure to determine and regulate the number of drops passing a drip chamber
Dispositif et procédé pour déterminer et régler le nombre de gouttes passant une chambre d'écoulement

(43) Veröffentlichungstag der Anmeldung: 21.11.1991
(73) Patentinhaber: Clinico Medical Electronics GmbH, D-99826 Mihla (DE)
(72) Erfinder:
(74) Vertreter: von Raffay, Vincenz, Dipl.-Ing.

(56) Entgegenhaltungen:
- EP-A- 0 110 685
- EP-A- 0 361 662
- US-A- 4 553 963
- US-A- 4 559 036

## Beschreibung

Die Erfindung betrifft eine Vorrichtung zum Ermitteln und Regeln der Anzahl von eine Tropfenkammer durchlaufenden Tropfen nach dem Oberbegriff des Hauptanspruches.

Eine derartige Vorrichtung ist aus der US-A-4,553,963 bekannt. Darüberhinaus gibt es Tropfenzähler mit Digitalanzeige, die die tatsächliche Tropfenanzahl pro Zeiteinheit angibt (vgl. hierzu beispielsweise das DE-U-84 32 849 und EP-A-361 662).
Nachteilig hat sich jedoch bei der Vorrichtung gemäß obiger US-A-4,553,963 bemerkbar gemacht, daß die Verbindung zwischen Rollklemmenrad und Motor mittels einem auf Wellen gelagerten Antriebsriemen aufgrund von Reibung verschleiß- und schmutzanfällig ist und deshalb einer exakten, kontinuierlichen Tropfenregulierung entgegenwirkt.

Der vorliegenden Erfindung liegt deshalb die Aufgabe zugrunde, die eingangs genannte Vorrichtung derart zu verbessern, daß neben der Überwachung der Tropfenzahl eine exakte, kontinuierliche Tropfenregulierung möglich ist, die personalungebunden automatisch abläuft und somit auch eine Feindosierung zuläßt, ohne daß antriebsmäßige Probleme auftreten.

Diese Aufgabe wird durch die im Anspruch 1 gekennzeichnete Vorrichtung überraschenderweise gelöst. Erfindungsgemäß wird also ein Regelgerät vorgesehen, das eine automatische, lineare Auf- und Abbewegung des darin aufgenommenen Rollklemmenrades durch einen Motor mit Spindelantrieb dann hervorruft, wenn eine Abweichung zwischen eingestellter und tatsächlich gemessener Tropfenanzahl festgestellt ist. Der Spindelantrieb stellt dabei erfindungsgemäß eine sichere, rutschfreie Verbindung zum Rollklemmenrad her und zwar über eine halbmondförmige Ausnehmung am Führungssegment, von der das Rollklemmenrad in etwa zur Hälfte aufgenommen wird. Es dürfte einleuchten, daß der Tropfenzähler mit dem Regelgerät elektrisch verbunden ist, das eine entsprechend geeignete Mikroprozessorschaltung aufweist.

Werden vom Regelgerät Abweichungen zwischen dem Soll- und Ist-Wert der Tropfenanzahl festgestellt, wird automatisch der Spindelantrieb betätigt, wodurch das in der Ausnehmung des Führungssegmentes angeordnete Rollklemmenrad nach oben oder unten bewegt wird, was zu einer Reduzierung oder Steigerung der Tropfengeschwindigkeit bis zu seinem Ausgleich von Soll- und Ist-Werten der Tropfenanzahl führt.

Weitere Vorteile und Merkmale gehen aus den Unteransprüchen hervor.

Im folgenden wird ein bevorzugtes Ausführungsbeispiel anhand der Zeichnung näher erläutert.

Es zeigt:
- Bild 1:: eine perspektivische Schemaansicht durch die erfindungsgemäße Vorrichtung; und
- Bild 2:: eine perspektivische Schemaansicht von Einzelteilen der in Bild 1 gezeigten Vorrichtung.

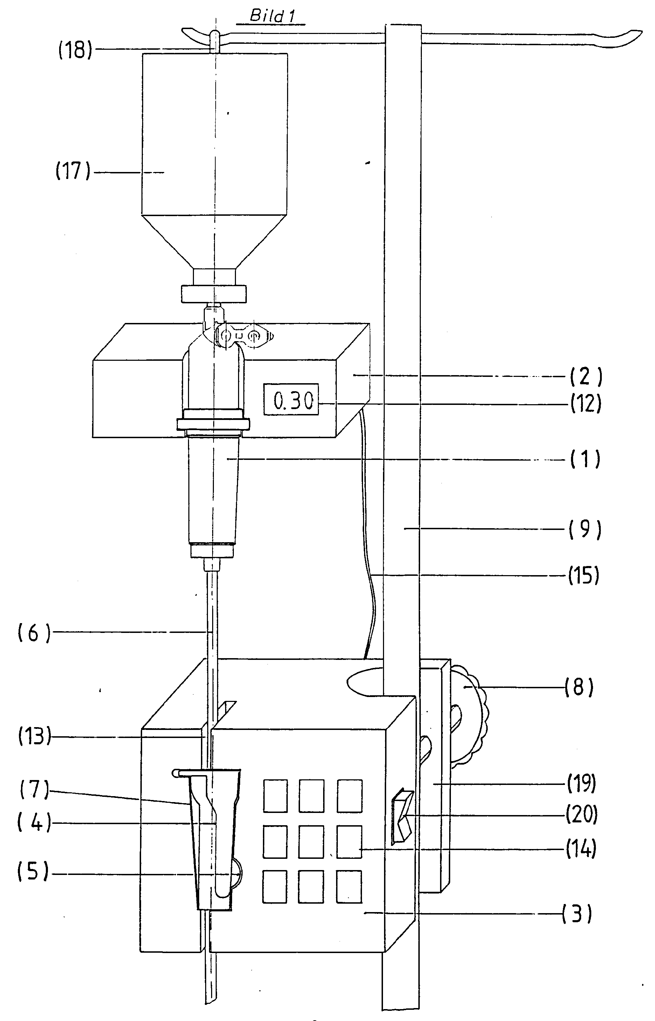

In Bild 1 ist ein Infusionsständerrohr 9 gezeigt, an dem oben links ein Infusionsbehälter 17 mit einer entsprechenden Infusionslösung aufgehängt (18) ist. Dieser Infusionsbehälter 17 steht in unmittelbarer Verbindung mit einer Tropfenkammer 1, die einen üblichen, zweiteiligen Aufbau besitzt, von dem die obere Hälfte in einen üblichen Tropfenzähler 2 mit Digitalanzeige 12 eingesetzt ist. Am unteren Ende der Tropfenkammer 1 ist ein Infusionsschlauch 6 angeordnet, der durch das Rollklemmengehäuse 4 hindurchverläuft, welches ein Rollklemmenrad 5 aufweist. Die Funktionsweise der marktüblichen Rollklemmen ist hinlänglich bekannt. Ihr Aufbau ist ebenfalls im wesentlichen gleich bzw. ähnlich.

Von besonderer erfindungsgemäßer Bedeutung ist es, daß der Schlauch 6 durch eine Bohrung bzw. Schlitz 13 im Gehäuse 3 des Regelgerätes hindurchverläuft, und daß das Rollklemmengehäuse 4 mit Rad 5 in die erweiterte Ausnehmung 7 im Gehäuse 3 eingesetzt wird. Der Einsatz ist paßgerecht bzw. in etwa formschlüssig, so daß eine sichere Halterung von Rollklemmengehäuse 4 mit Rad 5 gewährleistet ist. Besonders hervorzuheben ist, daß die Rollklemme 4,5 nur in exakt geschlossener Funktionsstellung in das Gehäuse 3 des Regelgerätes vom Bedienerpersonal eingelegt und entnommen werden kann. Ein besonderer Vorteil liegt darin, daß in das Gehäuse 3 praktisch alle marktüblichen Rollklemmen 4 mit Rad 5 eingelegt werden können, so daß eine universelle Einsatzmöglichkeit für Infusions- bzw. Transfusionsgeräte gegeben ist und somit eine wirtschaftliche und funktionelle Alternative zu den bekannten Infusionspumpen geschaffen wird.

Ferner geht aus Bild 1 hervor, daß das Regelgerätgehäuse 3 eine zweite Bohrung 19 aufweist, die von der ersten beabstandet und an der Außenseite angeordnet ist. Durch diese Bohrung hindurch verläuft das Infusionsständerrohr 9, an dem das Gehäuse 3 mittels der Schraube 8 befestigt wird. Das Gehäuse 3 weist einen Ein-und Ausschalter 20 sowie eine Tastatur 14 zur Eingabe der gewünschten Tropfenanzahl/Zeiteinheit (Sollwert) auf. Ein elektrisches Kabel 15 verbindet das einen Mikroprozessor (nicht gezeigt) aufweisende Regelgerät mit dem Tropfenzähler 2.

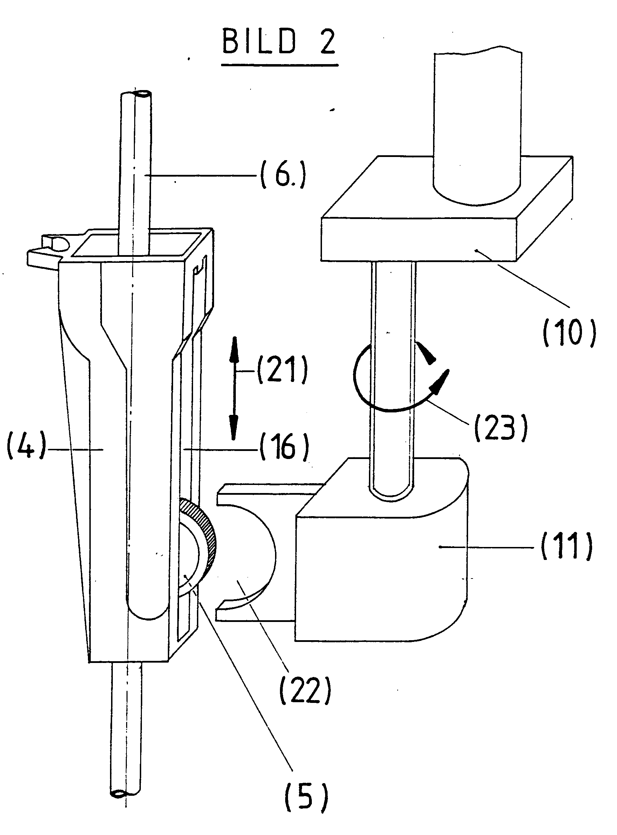

Die oben erwähnte Aufgabe der eigentlichen Regelung/Steuerung bzw. Überwachung der über die Tastatur 14 des Regelgerätes eingegebenen und mittels Mikroprozessor gespeicherten Soll-Tropfenanzahl wird in mechanischer Hinsicht durch die im Bild 2 schematisch im Ausschnitt dargestellten Details gelöst. Die linke Bildhälfte zeigt das stark vergrößerte Rollklemmengehäuse 4 mit Rollklemmenrad 5, welches sich im Gehäuseschlitz 16 auf- und abbewegen kann, wie dieses durch den Pfeil 21 angedeutet ist. Bekanntermaßen wird der Querschnitt des Schlauches 6 geringfügig durch das Drehen des Rades 5 verändert, so daß hierdurch die Tropfengeschwindigkeit bzw. die Menge der zu applizierenden Flüssigkeit pro Zeiteinheit geändert wird. In der rechten Hälfte des Bildes 2 ist der Spindelantrieb schematisch mit 10 und das damit verbundene Führungselement mit 11 bezeichnet. Dieses weist auf seiner linken Seite eine halbmondförmige Ausnehmung auf, die dem Durchmesser des Rades 5 entspricht und ebenfalls seine Breite besitzt. Das Rollklemmengehäuse 4 mit Rad 5 wird derart in die Ausnehmung 7 eingesetzt, daß das Rollklemmenrad 5 in etwa zur Hälfte von der Ausnehmung 22 aufgenommen wird. Der Spindelantrieb 10 ist durch den Pfeil 23 angedeutet und ermöglicht die Auf- oder Abbewegung des Führungselementes 11, respektive des damit verbundenen Rades 5.

Wird nun im Gebrauchszustand der erfindungsgemäßen Vorrichtung eine Abweichung zwischen dem Soll- und Istwert der Tropfenanzahl festgestellt, wird durch den nichtgezeigten Mikroprozessor im Gehäuse 3 des Regelgerätes entsprechend eine Auf- oder Abbewegung des Rollklemmenrades 5 über das Führungssegment 11 hervorgerufen, und zwar solange, bis ein Ausgleich zwischen dem Soll- und Istwert erzielt ist. Hierdurch wird eine kontinuierliche, automatische und genaue Steuerung bzw. Regelung für die unterschiedlichsten Infusions- oder Transfusionsmedien gewährleistet, ohne daß ein manuelles Eingreifen erforderlich wird.

## Patentansprüche

1. Vorrichtung zum Ermitteln und Regeln der Anzahl von eine Tropfenkammer (1) durchlaufenden Tropfen, mit einem Tropfenzähler (2) und einem an die Tropfenkammer (1) angeschlossenen Schlauch (6), der ein Rollklemmengehäuse (4) durchläuft, welches ein Rollklemmenrad (5) besitzt, sowie mit
einem Regelgerät mit Gehäuse (3), welches einen Schlitz (13) mit einer erweiterten Ausnehmung (7) aufweist, in die im Gebrauchszustand das Rollklemmengehäuse (4) mit Rollklemmenrad (5) eingesetzt ist, während der Schlauch (6) von dem Schlitz aufgenommen wird, und wobei das Rollklemmenrad (5) an seinem aus dem Gehäuseschlitz (16) des Rollklemmengehäuses herausragenden Teil formschlüssig von einem im Gehäuse (3) auf- und abbewegbaren Führungssegment (11) aufgenommen wird, das mit einem Antrieb (10) verbunden ist, und wobei das Regelgerät eine Tastatur (14) zum Einstellen der Tropfengeschwindigkeit bzw. Soll-Tropfenanzahl/Zeiteinheit und ein elektrisches Kabel (15) besitzt, mit dem es an den Tropfenzähler (2) angeschlossen ist, und wobei die Soll-Tropfen-Anzahl/ Zeiteinheit in einem vom Regelgerät (3) aufgenommenen Mikroprozessor gespeichert wird, der bei Abweichung gegenüber der tatsächlichen Tropfenanzahl den Antrieb betätigt, was zu einer Auf- oder Abbewegung vom Führungssegment und somit zu einer Reduzierung oder Steigerung der Tropfengeschwindigkeit bzw. der zu applizierenden Flüssigkeitsmenge/Zeiteinheit und zum Ausgleich von Soll- und Ist-Werten der Tropfenanzahl führt, dadurch gekennzeichnet, daß eine die Tropfenanzahl angebende Digitalanzeige (12) vorgesehen ist, und daß der Schlitz (13) in senkrechter Richtung verläuft, was zu einer senkrechten Auf- und Abbewegung vom Führungssegment (11) führt, und daß das Führungssegment (11) einen Spindelantrieb (10) und eine halbmondförmige Ausnehmung (22) aufweist, von der das Rollklemmenrad (5) in etwa zur Hälfte aufgenommen wird.

2. Vorrichtung nach Anspruch 1, dadurch gekennzeichnet, daß eine zweite (19) von der ersten (13) beabstandete Bohrung in senkrechter Richtung im Gehäuse (3) vorgesehen ist, in die eine Klemmschraube (8) zur Befestigung des Gehäuses (3) an einem Infusionsständer (9) einführbar ist.

3. Vorrichtung nach Anspruch 1 bis 1, dadurch gekennzeichnet, daß das Rollklemmengehäuse (4) mit seinem Rad (5) in etwa formschlüssig oder paßgerecht in die erweiterte Ausnehmung (7) eingesetzt ist.

4. Verfahren zum Ermitteln und Regeln der Anzahl von eine Tropfenkammer durchlaufenden Tropfen unter Verwendung der Vorrichtung nach einem oder mehreren der vorstehenden Ansprüche 1 bis 3, gekennzeichnet durch folgende Schritte:
a) Einstellen der gewünschten Tropfenanzahl/Zeiteinheit (Soll-Wert)
b) Ermitteln der tatsächlichen Tropfenanzahl/Zeiteinheit (Ist-Wert)
c) Feststellen von etwaigen Abweichungen zwischen Soll- und Ist-Wert
d) Hervorrufen einer automatischen Auf- oder Abbewegung des Rollklemmenrades unter Ausgleich von Soll- und Ist-Werten.

## Claims

1. Apparatus for determining and regulating the number of drops passing through a drop chamber (1), with a drop counter (2) and a tube (6) connected to the drop chamber (1), which passes through a roll clamp casing (4) having a roll clamp wheel (5), as well as with a regulating device having a casing (3), which has a slot (13) with a widened recess (7), in which the roll clamp casing (4) with the roll clamp wheel (5) is inserted in the use state, whilst the tube (6) is received by the slot and in which the roll clamp wheel (5) is positively received with its part projecting out of the roll clamp casing slot (16) by a guide segment (11) moving up and down within the casing (3) and which is connected to a drive (10) and in which the regulating device has a keyboard (14) for setting the drop speed or the desired number of drops per unit of time and an electric cable (15) by which it is connected to the drop counter (2) and in which the desired number of drops per unit of time is stored in a microprocessor received by the regulating device (3), which in the case of a divergence compared with the actual number of drops operates the drive, which leads to an upward or downward movement of the guide segment and consequently to a reduction or an increase in the drop speed or the liquid quantity to be applied per time unit and to a balancing of the desired and actual number of drops, characterized in that a digital read-out (12) giving the number of drops is provided and that the slot (13) runs vertically, which leads to a vertical upward and downward movement of the guide segment (11) and that the guide segment (11) has a spindle drive (10) and a crescent-shaped recess (22), which receives approximately half of the roll clamp wheel (5).

2. Apparatus according to claim 1, characterized in that there is a second bore (19) spaced from the first bore (13) in the vertical direction within the casing (3) and into it can be inserted a setscrew (8) for fixing the casing (3) to an infusion stand (9).

3. Apparatus according to claim 1, characterized in that the roll clamp casing (4) is inserted by its wheel (5) in an approximately positive or precise fitting manner in the widened recess (7).

4. Method for determining and regulating the number of drops passing through a drop chamber using the apparatus according to one or more of the claims 1 to 3, characterized by the following steps:
a) setting the desired number of drops per unit of time (desired value),
b) determining the actual number of drops per unit of time (actual value),
c) determining any divergences between the desired and actual values,
d) bringing about an automatic upward or downward movement of the roll clamp wheel whilst balancing the desired and actual values.

## Revendications

1. Dispositif de détermination et de régulation du nombre de gouttes traversant une chambre à gouttes (1), avec un compteur à gouttes (2) et un tuyau (6), raccordé à la chambre à gouttes (1), traversant un carter de serrage par roulement (4), comportant une roue de serrage à roulement (5), ainsi qu'un appareil de régulation comportant un carter (3), présentant une fente (13) avec un évidement (7) agrandi, dans lequel, à l'état d'utilisation, le carter de serrage à roulement (4) est utilisé, avec la roue de serrage à roulement (5), tandis que le tuyau (6) est reçu par la fente, et la roue de serrage à roulement (5) est reçue, sur sa partie faisant saillie hors de la fente (16) du carter à serrage à roulement, avec ajustement de forme, par un segment de guidage (11), levable et abaissable dans le carter (3) et relié à un entraînement (10), et l'appareil de régulation comportant un clavier (14), pour régler la vitesse d'égouttement, respectivement le nombre de gouttes de consigne/unité de temps et comportant un cable électrique (15), à l'aide duquel il est raccordé au compteur de gouttes (2), et le nombre de gouttes de consigne/unité de temps étant stocké en mémoire dans un microprocesseur logé dans l'appareil de régulation (3) et actionnant l'entraînement en cas d'écart par rapport au nombre de gouttes effectif, ce qui mène à un mouvement de levée ou d'abaissement du segment de guidage et ainsi à une diminution ou une augmentation de la vitesse d'égouttement, respectivement de la quantité de liquide/unité de temps à appliquer et à l'équilibre entre les valeurs de consigne et réelles du nombre de gouttes, caractérisé en ce qu'est prévu une indication numérique (12), indiquant le nombre de gouttes, et en ce que la fente (13) s'étend en direction verticale, ce qui mène à guidage (11) orienté verticalement, et en ce que le segment de guidage (11) présente un entraînement à tige filetée (10) et un évidement (22) en forme de demi-lune, qui loge à peu près la moitié de la roue de serrage à roulement (5).

2. Dispositif selon la revendication 1, caractérisé en ce qu'est prévu, dans la direction verticale, dans le carter (3), un deuxième (19) perçage, espacé du premier (13), dans lequel une vis de serrage (8) est susceptible d'être introduite, en vue de fixer le carter (3) sur un support d'infusion (9).

3. Dispositif selon les revendications 1 et 2, caractérisé en ce que le carter de serrage à roulement(4) est inséré, avec sa roue (5), **avec** ajustement de forme ou exactement ajusté , dans l'évidement (7) agrandi.

4. Procédé de détermination et de régulation du nombre de gouttes traversant une chambre à gouttes, utilisant le dispositif selon l'une ou plusieurs des revendications 1 à 3 ci-dessus, caractérisé par les étapes suivantes :
a) réglage de la valeur souhaitée du nombre de gouttes/ unité de temps (valeur de consigne)
b) détermination da la valeur effective du nombre de gouttes/unité de temps (valeur réelle)
c) constatation des écarts résiduels entre valeur de consigne et valeur réelle,
d) commande d'un mouvement de levée ou d'abaissement automatique de la roue de serrage à roulement, en opérant une égalisation entre la valeur de consigne et la valeur réelle.
